# EUROPÄISCHE PATENTSCHRIFT

(11) **EP 0 550 861 B1**
(45) Veröffentlichungstag und Bekanntmachung des Hinweises auf die Patenterteilung: **09.04.1997**
(21) Anmeldenummer: 92121547.1
(22) Anmeldetag: 18.12.1992
(51) Int. Cl.: B01J 23/94

(54) **Verfahren zur Regenerierung von Metalloxid-Katalysatoren**
Process for regenerating metal oxide containing catalysts
Procédé pour la régénération des catalyseurs contenant oxyde métallique

(30) Priorität: 08.01.1992 DE 4200248
(43) Veröffentlichungstag der Anmeldung: 14.07.1993
(73) Patentinhaber: BASF Aktiengesellschaft, 67063 Ludwigshafen (DE)
(72) Erfinder: Karrer, Lothar, Dr., W-6102 Pfungstadt (DE); Herzog, Klaus, Dr., W-6700 Ludwigshafen (DE); Aichinger, Heinrich, Dr., W-6800 Mannheim 1 (DE); Eichhorn, Hans-Dieter, Dr., Cleveland TS 199 TN (GB); Herrmann, Guenter, Dr., W-6900 Heidelberg (DE)

(56) Entgegenhaltungen:
- EP-A- 0 452 630
- DD-A- 204 409
- DE-C- 2 848 850
- DATABASE WPIL Derwent Publications Ltd., London, GB; AN 82-11345E

## Beschreibung

Gegenstand der Erfindung ist ein Verfahren zur Regenerierung von Metalloxid-Katalysatoren mit verminderter Aktivität, die mindestens eines der Elemente Antimon, Eisen, Bismut, Molybdän, Vanadium, Wolfram und/oder Uran sowie peptisierbare Träger enthalten.

Bei der Oxidation von Propen zu Acrolein oder der Amoxidation von Propen zu Acrylnitril in Gegenwart von Metalloxid-Katalysatoren im Wirbelbett verlieren diese im Verlauf der Reaktion einen Teil ihrer Aktivität, werden durch die mechanische Beanspruchung zerkleinert, so daß deren Wirbelfähigkeit beeinträchtigt wird. Ein Teil wird mit den Reaktionsgasen ausgetragen und wird mit organischen und anorganischen Bestandteilen verunreinigt erhalten.

Aus der DE-C 25 60 480 ist ein Verfahren zur Regenerierung von Metalloxid-Katalysatoren mit verringerter katalytischer Aktivität bekannt, die ein Oxid des Antimons und mindestens eines der Metalle Eisen, Cobalt, Nickel, Mangan, Uran, Zink und Kupfer und als zusätzliche Komponente eines der Oxide von Molybdän, Vanadium und/oder Wolfram sowie Telluroxid enthalten, wobei man den zu regenerierenden Katalysator mit einer wäßrigen Lösung oder Suspension imprägniert, die eine Molybdän-, Vanadium- und/oder Wolfram-Verbindung sowie eine Tellurverbindung enthält und anschließend trocknet und bei 400 bis 850°C calciniert. Dieses Verfahren hat den Nachteil, daß ein feinteiliger und nicht wirbelfähiger Katalysator durch die Regenerierung erhalten wird und die Festigkeit des Katalysators gering ist.

Nach einem anderen in der DE-C 28 48 850 beschriebenen Verfahren wird ein gebrauchter Metalloxid-Katalysator, der Antimon sowie mindestens eines der Metalle Eisen, Cobalt, Nickel, Mangan, Uran, Cer, Zinn und/oder Kupfer enthält, mit einer wäßrigen Salpetersäure-Lösung und/oder einer wäßrigen Lösung von Ammoniumnitrat, einem Alkali- oder Erdalkalinitrat oder einem Nitrat von Metallen wie Eisen, Cobalt, Nikkel, Kupfer, Bismut und/oder Tellur imprägniert und dann getrocknet und erhitzt. Auch nach diesem Verfahren ist aus einem verbrauchten mechanisch beschädigten Katalystor kein wirbelfähiger Katalysator erhältlich.

Nach einem weiteren in den DD-Patentschriften 371 997 und 204 409 beschriebenen Verfahren wird ein verbrauchter Katalysator, der eines der Oxide von Bismut, Molybdän, Eisen und/oder Chrom sowie ggf. weitere Elemente enthält, regeneriert, indem man den gebrauchten Katalysator zunächst auf eine Teilchengröße unter 50 µm mahlt und ihn danach mit einer wäßrigen Salzlösung oder Aufschlammung oxidischer Verbindungen mindestens eines der Elemente Bismut, Molybdän, Eisen und/oder Chrom als Reaktivierungslösung bei einem pH-Wert von 0 bis 3 und einer Temperatur von 20 bis 100°C behandelt, wobei die Reaktivierungslösung so bemessen ist, daß zugleich die fünffache Menge an Frischkatalysator erzeugt wird, anschließend das Gesamtgemisch sprühgetrocknet und getempert wird. Das Verfahren hat den Nachteil, daß je Gewichtsteil verbrauchten Katalysator 5 Teile neuer Katalysator hergestellt werden müssen. Es ist kein Hinweis zu entnehmen, wie aus einem verbrauchten Katalysator allein wiederum ein wirbelfähiger Katalysator mit guter mechanischer Festigkeit erhältlich ist.

Es war deshalb die technische Aufgabe gestellt, regenerierte Metalloxid-Katalysatoren zur Verfügung zu stellen, die wirbelfähig sind, hohe Abriebfestigkeit und Bruchfestigkeit aufweisen, einen hohen Umsatz an Propen und hohe Selektivität zu Wertprodukten ermöglichen.

Diese Aufgabe wird gelöst in einem Verfahren zur Regenerierung von Metalloxid-Katalysatoren mit verminderter Aktivität, die mindestens eines der Elemente Antimon, Eisen, Bismut, Molybdän, Vanadium, Wolfram und/oder Uran sowie peptisierbare Träger enthalten, welches folgende Schritte umfaßt:
a) Mahlen des Metalloxid-Katalysators unter Mitverwendung von Wasser unter Erhalt einer wäßrigen Suspension des Metalloxid-Katalysators mit einer Teilchengröße von 10 nm bis 10 µm,
b) Erhitzen der wäßrigen Suspension des Metalloxid-Katalysators aus der Stufe a) auf eine Temperatur von 30 bis 120°C und Zugabe einer Säure, die den Katalysatorträger peptisiert,
c) Sprühtrocknen der sauren Suspension des Metalloxid-Katalysators aus der Stufe b) unter Erhalt wirbelfähiger Metalloxid-Katalysatorteilchen,
d) Erhitzen der wirbelfähigen Metalloxid-Katalysatorteilchen aus der Stufe c) auf eine Temperatur von 500 bis 850°C unter Erhalt eines wirbelfähigen Metalloxid-Katalysators.

Gegenstand der Erfindung sind auch regenerierte Katalysatoren, die nach dem obengenannten Verfahren erhältlich sind.

Weiter ist ein Gegenstand der Erfindung die Verwendung regenerierter Metalloxid-Katalysatoren, die nach dem obengenannten Verfahren erhältlich sind, für die Amoxidation von Olefinen zu den entsprechenden Nitrilen, die Oxidation von Olefinen zu den entsprechenden Aldehyden oder die oxidative Dehydrierung von Olefinen zu Diolefinen, sowie die Amoxidation von methylsubstituierten Aromaten zu den entsprechenden Nitrilen.

Das neue Verfahren hat den Vorteil, daß die regenerierten Katalysatoren sich durch eine hohe Abriebfestigkeit und Bruchfestigkeit auszeichnen. Ferner hat das neue Verfahren den Vorteil, daß man regenerierte Katalysatoren erhält, die hohe Umsätze erlauben und eine hohe Selektivität zu den gewünschten Endprodukten ermöglichen. Ferner zeichnen sich die erfindungsgemäß hergestellten Katalysatoren durch ausgezeichnete Wirbelfähigkeit aus. Schließlich hat das neue Verfahren den Vorteil, daß die Entsorgung von verbrauchten Katalysatoren entfällt, da sie in wertvolle gebrauchsfähige Katalysatoren umgewandelt werden.

In der Stufe a) geht man erfindungsgemäß von Metalloxid-Katalysatoren mit verminderter Aktivität, die mindestens eines der Elemente Antimon, Eisen, Bismut, Molybdän, Vanadium, Wolfram und/oder Uran sowie peptisierbare Träger enthalten, aus. Solche Katalysatoren können auch weitere katalytisch aktive Elemente enthalten, z.B. Elemente der 1., 2., 3., 5. oder 6. Hauptgruppe sowie 5. bis 7. Nebengruppe des periodischen Systems. Geeignete peptisierbare Träger sind beispielsweise Aluminiumoxid und Siliciumdioxid. Besonders bevorzugt ist Siliciumdioxid, das in feiner Verteilung, z.B. ausgehend von einem Sol, im Katalysator vorliegt. Unter peptisierbar ist die Ausbildung von OH-Gruppen auf der Oberfläche eines Teilchens unter Einwirkung von wäßriger Säure zu verstehen, wobei die gebildeten OH-Gruppen befähigt sind, mit OH-Gruppen eines benachbarten Teilchens wiederum beim Trocknen und Glühen eine Bindung einzugehen und somit eine feste Bindung innerhalb eines größeren Verbandes entsteht. Vorteilhaft beträgt der Gehalt an peptisierbaren Trägern 20 bis 80 Gew.-%, bezogen auf die Summe von Träger und katalytisch aktiven Metalloxiden. In der Regel haben die verbrauchten Katalysatoren eine mittlere Teilchengröße von 1 bis 100 µm, insbesondere von 2 bis 25 µm.

Solche verbrauchten Katalysatoren fallen beispielsweise an bei der oxidativen Dehydrierung von Butan zu Buten, bei der oxidativen Dehydrierung von Propen zu Acrolein, ferner bei der Amoxidation von Propen und von methylsubstituierten Aromaten zu den entsprechenden Nitrilen, der oxidativen Dehydrierung von Olefinen bevorzugt in der Wirbelschicht.

Bevorzugte Katalysatoren sind beispielsweise Eisen-BismutMolybdänoxid-Katalysatoren, die gegebenenfalls mindestens eines der Elemente Nickel, Cobalt, Magnesium, Vanadium, Wolfram, Calcium, Phosphor, Arsen, Antimon und/oder ein Alkalimetall sowie gegebenenfalls zusätzlich mindestens eines der Elemente Zink, Cadmium, Bor, Wolfram, Yttrium, Zirkon, Silber, Schwefel, Cer, Thorium, Chrom, Zinn, Mangan, Indium, Kupfer, Tantal, Tellur und/oder Lanthan enthalten können. Geeignete Katalysatoren werden beispielsweise beschrieben in der deutschen Patentschrift 25 30 959.

Eine andere Art von bevorzugten Katalysatoren sind Antimon-Eisen-Tellur-Oxidkatalysatoren, die zusätzlich mindestens eines der Elemente Cobalt, Nickel, Mangan, Zinn und/oder Kupfer sowie mindestens eines der Metalle Molybdän, Vanadium und/oder Wolfram und ferner mindestens eines der Elemente Zink, Kalium, Magnesium, Aluminium, Zirkon, Bismut, Cer, Chrom, Phosphor und/oder Bor enthalten können. Geeignete Katalysatoren werden beispielsweise beschrieben in der deutschen Patentschrift 25 60 480.

Sofern Metalloxid-Katalysatoren regeneriert werden sollen, die zudem organische und anorganische Verunreinigung enthalten, wie sie z.B. im Quench der Reaktionsgase aus der Amoxidation anfallen, so ist es vorteilhaft, diese vor dem Mahlen mit Sauerstoff enthaltenden Gasen, zweckmäßig Luft, bei einer Temperatur von 300 bis 700°C zu tempern. Solche verunreinigten Metalloxid-Katalysatoren enthalten in der Regel 10 bis 50 % nicht näher definierbare organische Bestandteile, ferner gegebenenfalls 1 bis 20 % Ammoniumsulfat. Die oxidative Behandlung mit molekularen Sauerstoff enthaltenden Gasen, wie Luft, erfolgt vorteilhaft über einen Zeitraum von 1/2 bis 30 Stunden bei einer Temperatur von 300 bis 700°C, z.B. im Wirbelbett, im Drehrohrofen oder in einem Tunnelofen.

Die als Ausgangsstoffe verwendeten Metalloxid-Katalysatoren werden unter Mitverwendung von Wasser gemahlen. Vorteilhaft verwendet man je Gewichtsteil Metalloxid-Katalysator 0,5 bis 10 Gew.-Teile Wasser. Das Mahlen erfolgt vorteilhaft mit einer Rührwerkskugelmühle. Man erhält hierbei eine Suspension von Metalloxid-Katalysatoren in Wasser, wobei die Teilchengröße des gemahlenen Metalloxid-Katalysators von 10 nm bis 10 µm, insbesondere von 10 nm bis 2 µm beträgt. Besonders vorteilhaft ist die mittlere Teilchengröße von 50 nm bis 1 µm.

Zweckmäßig setzt man der wäßrigen Suspension von Metalloxid-Katalysatoren vor der weiteren Verarbeitung wäßrige Ammoniaklösung, z.B. mit einem Gehalt von 10 bis 80 Gew.-% NH₃ und/oder die Lösung eines Ammoniumsalzes mit einer zersetzbaren Säure, z.B. Salpetersäure, Essigsäure, Ameisensäure oder Oxalsäure, insbesondere Nitrat, zu. Vorteilhaft soll der Gehalt an NH₄⁺-Ionen pro Liter 10 bis 200 g betragen. Man hält vorteilhaft für einen Zeitraum von z.B. 15 bis 120 Minuten eine Temperatur von 30 bis 102°C ein.

In der Stufe b) werden der wäßrigen Suspension des Metalloxid-Katalysators aus der Stufe a) unter Erhitzen auf eine Temperatur von 30 bis 120°C mindestens eine Säure, die den Katalysatorträger zu peptisieren im Stande ist, zugesetzt.

Geeignete Säuren sind beispielsweise Schwefelsäure, Salpetersäure, Essigsäure, Ameisensäure, Glutarsäure oder Oxalsäure. Besonders bewährt hat sich Salpetersäure. Vorteilhaft wird soviel Säure zugesetzt, daß eine stark saure Lösung entsteht, z.B. mit einem pH-Wert von 0 bis 3. In der Regel wendet man je Gewichtsteil suspendierten Metalloxid-Katalysator 0,01 bis 10 Gew.-Teile Säure an. Vorteilhaft hält man eine Temperatur von 30 bis 120°C für einen Zeitraum von 15 bis 120 Minuten ein.

Vorteilhaft setzt man der Suspension in Stufe a) oder b) Metallsalze zu, z.B. Nitrate von mindestens einem der Elemente, die im Katalysator bereits enthalten sind und ergänzt werden sollen, in einer Menge, die zur Ergänzung ausreicht. Zweckmäßig werden beispielsweise zugesetzt Nitrate oder andere Verbindungen oder die Metalle selbst von Antimon, Eisen, Cobalt, Nickel, Mangan, Kupfer, Molybdän, Wolfram oder Tellur. Insbesondere werden die Mengen an flüchtigem Molybdän und Tellur ergänzt. Darüber hinaus ist es auch möglich, durch Zugabe von weiteren Elementen den Katalysator zusätzlich zu modifizieren, z.B. durch Zugabe von Lanthan, Cer, Chrom, Zink oder Phosphosäure, ferner Heteropolysäuren von Phosphorsäure, Kieselsäure und Wolframsäure in katalytisch wirksamen Mengen.

Sofern auf ein Zusatz von Ammoniak oder Ammoniumnitrat in der Stufe a) verzichtet wurde, setzt man vorteilhaft Ammoniumnitrat oder Salze anderer zersetzlicher Säuren wie Ameisensäure, Essigsäure oder Oxalsäure, insbesondere Ammoniumnitrat in der Stufe b) zu, vorteilhaft in einer Menge von 5 bis 500 g Ammoniumionen je Liter.

Es hat sich bewährt, wenn die Suspension vor der Weiterverarbeitung durch Zugabe von wäßrigem Ammoniak auf einen pH-Wert von 5 bis 0,5, vorteilhaft 0 bis 3, insbesondere von 1 bis 2,5 gebracht wird. Vorteilhaft rührt man die Suspension bei einer Temperatur von 40 bis 80°C noch weitere 15 bis 60 Minuten.

Die so aus der Stufe b) erhaltene sauere Suspension des Metalloxid-Katalysators wird in Stufe c) sprühgetrocknet und ein wirbelfähiger Vorläufer des Metalloxid-Katalysators erhalten. Vorteilhaft haben die erhaltenen Teilchen einen mittleren Durchmesser von 50 µm ± 20.

Die in der Stufe c) erhaltenen wirbelfähigen Metalloxid-Katalysator-Teilchen werden anschließend in der Stufe d) bei einer Temperatur von 500 bis 850°C calciniert, vorteilhaft für einen Zeitraum von 0,5 bis 10 Stunden. Besonders bewährt hat es sich, wenn man das Calcinieren in zwei Stufen vornimmt, z.B. in einer ersten Stufe von 0,5 bis 10 Stunden zur Zersetzung von Ammoniumsalzen z.B. bei einer Temperatur von 150 bis 400°C und in einer zweiten Stufe bei einer Temperatur von 500 bis 850°C.

Die nach der Erfindung erhältlichen regenerierten Metalloxid-Katalysatoren zeichnen sich durch eine hervorragende Abriebfestigkeit, z.B. von 0,5 bis 2 g/kg Kat./h in einer Strahlmühle aus.

Die nach der Erfindung erhältlichen Katalysatoren eignen sich für die oxidative Dehydrierung von Olefinen, weiter für die Herstellung von Acrolein aus Propen, insbesondere für die Amoxidation von Propen bzw. methylsubstituierten Aromaten zur dementspechenden Nitrilen.

Vorteilhaft wird die Amoxidation von Propen zu Acrylnitril durchgeführt durch Umsetzen von Propen mit molekularen Sauerstoff enthaltenden Gasen wie Luft im Überschuß sowie Ammoniak und gegebenenfalls Wasserdampf an einem fluidisierten Metalloxid-Katalysator wie oben aufgeführt bei einer Temperatur von 300 bis 500°C und unter erhöhtem Druck, z.B. von 1 bis 4 bar.

Der Gegenstand der Erfindung sei an folgenden Beispielen veranschaulicht.

### Beispiel 1

Ein ausgeschleuster Katalysator der Zusammensetzung Fe₁₀Sb₂₅Te_{1,0}W_{0,25}O₆₈(SiO₂)₃₀ der 40 Gew.-% organische Anteile und 10 Gew.-% Ammonsulfat enthielt mit einer Teilchengröße von zwei Drittel der Masse mit einem Durchmesser von ca. < 16 µm und einem Drittel der Masse von Bestandteilen > 16 µm wurde in einem Drehrohr für einen Zeitraum von 5 Stunden unter Zufuhr von Luft auf eine Temperatur von 500°C erhitzt. Der so erhaltene Katalysator hatte einen Anteil von organischen Bestandteilen und Ammoniumsulfat von weniger als 1 Gew.-%.

Der so vorbehandelte Metalloxid-Katalysator wurde mit 1,5 Teilen Wasser versetzt und in einer Rührwerksmühle auf eine mittlere Partikelgröße von 550 nm zerkleinert. Die so erhaltene Suspension wurde je Gewichtsteil Katalysator mit 2 Gew.-teilen einer 23 gew.-%igen wäßrigen Salpetersäure versetzt, auf 50°C erhitzt und für 30 Minuten gerührt. Anschließend wurde mit wäßriger Ammoniaklösung die Suspension auf ein pH-Wert von 2 eingestellt und bei einer Temperatur von 50°C noch weitere 30 Minuten gerührt.

Anschließend wurde die so erhaltene Suspension sprühgetrocknet und anschließend bei einer Temperatur von 720°C für 3 Stunden calciniert. Die erhaltenen Katalysatorteilchen hatten einen mittleren Durchmesser von 50 µm.

1000 Gew.-teile des so erhaltenen Katalysators wurden in einem Wirbelbett stündlich mit 6350 Vol.-teilen (bezogen auf Normbedingungen) einer Gasmischung aus Propen, Ammoniak und Luft beaufschlagt. Die Gasmischung hatte ein molares Verhältnis von Luft zu Propen von 12 und ein molares Verhältnis von Ammoniak zu Propen 1,2. Die Temperatur im Katalysatorwirbelbett beträgt 460°C, der Druck 1 bar. Das aus dem Reaktor austretende Gas wurde gaschromatographisch untersucht und die Selektivität der Acrylnitrilbildung und der Propenumsatzgrad bestimmt. Der Umsatz am Propen beträgt 98 %, die Ausbeute an Acrylnitril 77,5 %. Die Katalysatorteilchen sind rund und abriebfest wie neuer Katalysator. Der Durchmesser der Katalysatorteilchen hat sich lediglich von 51 µm auf 50 µm verändert.

### Beispiel 2

Man verfährt wie im Beispiel 1 beschrieben, mit dem einzigen Unterschied, daß der Katalysator nicht gemahlen wird. Die erhaltenen Katalysatorteilchen haben keine runde Form und einen Durchmesser von 62 µm. Der Katalysator zerfiel im Wirbelbett rasch zu Feinstaub und war nicht verwendbar.

## Patentansprüche

1. Verfahren zur Regenerierung von Metalloxid-Katalysatoren mit verminderter Aktivität, die mindestens eines der Elemente Antimon, Eisen, Bismut, Molybdän, Vanadium, Wolfram und/oder Uran und ein peptisierbaren Träger enthalten, welches folgende Schritte umfaßt:
a) Mahlen des Metalloxid-Katalysators unter Mitverwendung von Wasser unter Erhalt einer wäßrigen Suspension des Metalloxid-Katalysators mit einer Teilchengröße von 10 nm bis 10 µm,
b) Erhitzen der wäßrigen Suspension des Metalloxid-Katalysators aus der Stufe a) auf eine Temperatur von 30 bis 120°C und Zugabe einer Säure, die den Katalysatorträger peptisiert,
c) Sprühtrocknen der sauren Suspension des Metalloxid-Katalysators aus der Stufe b) unter Erhalt wirbelfähiger Metalloxid-Katalysatorteilchen,
d) Erhitzen der wirbelfähigen Metalloxid-Katalysatorteilchen aus der Stufe c) auf eine Temperatur von 500 bis 850°C unter Erhalt eines wirbelfähigen Metalloxidkatalysators.

2. Verfahren nach Anspruch 1, dadurch gekennzeichnet, daß man in Stufe a) den Metalloxid-Katalysator auf eine mittlere Teilchengröße von 10 bis 1000 nm zerkleinert.

3. Verfahren nach den Ansprüchen 1 und 2, dadurch gekennzeichnet, daß man der Suspension in Stufe (a) oder (b) wäßrigen Ammoniak oder Ammoniumnitrat zusetzt.

4. Verfahren nach den Ansprüchen 1 bis 3, dadurch gekennzeichnet, daß man in der Stufe b) der Metalloxid-Katalysator-Suspension Salpetersäure zusetzt.

5. Verfahren nach den Ansprüchen 1 bis 4, dadurch gekennzeichnet, daß man in der Stufe b) je Gewichtsteil Metalloxid-Katalysator 0,01 bis 10 Gew.-teile Säure zusetzt.

6. Verfahren nach den Ansprüchen 1 bis 5, dadurch gekennzeichnet, daß man in Stufe a) oder b) zusätzlich mindestens eines der Elemente Mo, Sb oder Te zusetzt.

7. Verfahren nach den Ansprüchen 1 bis 6, dadurch gekennzeichnet, daß man anschließend an Stufe b) zu der wäßrigen Suspension Ammoniak zusetzt bis zu einem pH-Wert von 5 bis 0,5.

8. Verfahren nach den Ansprüchen 1 bis 7, dadurch gekennzeichnet, daß der Metalloxid-Katalysator Siliciumdioxid und/oder Aluminiumoxid als Träger enthält.

9. Verfahren nach den Ansprüchen 1 bis 8, dadurch gekennzeichnet, daß man einen Metalloxid-Katalysator verwendet, der Oxide von Antimon, Eisen und Tellur sowie mindestens eines der Elemente Eisen, Molybdän, Wolfram, Vanadium oder Kupfer enthält.

10. Verfahren nach den Ansprüchen 1 bis 8, dadurch gekennzeichnet, daß man einen Metalloxid-Katalysator verwendet, der Oxide von Bismut, Molybdän, Eisen enthält, sowie mindestens eines der Elemente K, Rb, Cs, Cr, Mn oder P.

11. Verfahren nach den Ansprüchen 1 bis 10, dadurch gekennzeichnet, daß man den Metalloxid-Katalysator vor der Stufe a) unter Zufuhr von molekularen Sauerstoff enthaltenden Gasen auf eine Temperatur von 300 bis 700°C erhitzt.

## Claims

1. A process for regenerating deactivated metal oxide catalysts comprising at least one of the elements antimony, iron, bismuth, molybdenum, vanadium, tungsten and/or uranium and a peptizable carrier, comprising the steps of:
a) grinding the metal oxide catalyst in the presence of water to obtain an aqueous suspension of the metal oxide catalyst having a particle size of from 10 nm to 10 µm,
b) heating the aqueous suspension of the metal oxide catalyst of stage a) to 30 - 120°C and adding an acid which peptizes the carrier,
c) spray drying the acidic suspension of the metal oxide catalyst of stage b) to obtain fluidizable metal oxide catalyst particles,
d) heating the fluidizable metal oxide catalyst particles of stage c) to 500 - 850°C to obtain a fluidizable metal oxide catalyst.

2. A process as claimed in claim 1, wherein, in stage a), the metal oxide catalyst is comminuted to an average particle size of from 10 to 1000 nm.

3. A process as claimed in either of claims 1 and 2, wherein aqueous ammonia or ammonium nitrate is added to the suspension in stage (a) or (b).

4. A process as claimed in any of claims 1 to 3, wherein nitric acid is added in stage b).

5. A process as claimed in any of claims 1 to 4, wherein per part by weight of metal oxide catalyst from 0.01 to 10 parts by weight of acid are added in stage b).

6. A process as claimed in any of claims 1 to 5, wherein additionally at least one of the elements Mo, Sb or Te is added in stage a) or b).

7. A process as claimed in any of claims 1 to 6, wherein, following stage b), ammonia is added to the aqueous suspension to a pH of from 5 to 0.5.

8. A process as claimed in any of claims 1 to 7, wherein the metal oxide catalyst contains silicon dioxide and/or aluminum oxide as carrier.

9. A process as claimed in any of claims 1 to 8, wherein the metal oxide catalyst used contains the oxides of antimony, iron and tellurium and also at least one of the elements iron, molybdenum, tungsten, vanadium or copper.

10. A process as claimed in any of claims 1 to 8, wherein the metal oxide catalyst used contains the oxides of bismuth, molybdenum and iron and also at least one of the elements K, Rb, Cs, Cr, Mn or P.

11. A process as claimed in any of claims 1 to 10, wherein, prior to stage a), the metal oxide catalyst is heated to 300 - 700°C under a gas containing molecular oxygen.

## Revendications

1. Procédé pour la régénération de catalyseurs à oxyde métallique avec une activité réduite, qui contiennent au moins un des éléments antimoine, fer, bismuth, molybdène, vanadium, tungstène et/ou uranium et un support peptisable, qui comprend les étapes suivantes :
a) le broyage des catalyseurs à oxyde métallique avec l'utilisation conjointe d'eau de façon à obtenir une suspension aqueuse du catalyseur à oxyde métallique avec une granulométrie de 10 nm à 10 µm,
b) le chauffage de la suspension aqueuse du catalyseur à oxyde métallique de l'étape a) à une température de 30 à 120 °C et l'addition d'un acide qui peptise le support du catalyseur,
c) le séchage par pulvérisation de la suspension acide du catalyseur à oxyde métallique de l'étape b) de façon à obtenir des particules de catalyseur à oxyde métallique fluidisables,
d) le chauffage des particules de catalyseur à oxyde métallique fluidisables de l'étape c) à une température de 500 à 850 °C de façon à obtenir un catalyseur à oxyde métallique fluidisable.

2. Procédé selon la revendication 1, caractérisé en ce qu'on réduit à l'étape a) le catalyseur à oxyde métallique à une granulométrie moyenne de 10 à 1000 nm.

3. Procédé selon les revendications 1 et 2, caractérisé en ce qu'on ajoute de l'ammoniaque ou du nitrate d'ammonium à la suspension à l'étape (a) ou (b).

4. Procédé selon les revendications 1 à 3, caractérisé en ce qu'on ajoute de l'acide nitrique à la suspension de catalyseur à oxyde métallique à l'étape b).

5. Procédé selon les revendications 1 à 4, caractérisé en ce qu'on ajoute 0,01 à 10 parts en poids d'acide par part en poids de catalyseur à oxyde métallique à l'étape b).

6. Procédé selon les revendications 1 à 5, caractérisé en ce qu'on ajoute en outre au moins un des éléments Mo, Sb ou Te à l'étape a) ou b).

7. Procédé selon les revendications 1 à 6, caractérisé en ce qu'on ajoute de l'ammoniac à la suspension aqueuse à la suite de l'étape b) jusqu'à un pH de 5 à 0,5.

8. Procédé selon les revendications 1 à 7, caractérisé en ce que le catalyseur à oxyde métallique contient du dioxyde de silicium et/ou de l'oxyde d'aluminium à titre de support.

9. Procédé selon les revendications 1 à 8, caractérisé en ce qu'on utilise un catalyseur à oxyde métallique qui contient des oxydes d'antimoine, de fer et de tellure, de même qu'au moins un des éléments fer, molybdène, tungstène, vanadium ou cuivre.

10. Procédé selon les revendications 1 à 8, caractérisé en ce qu'on utilise un catalyseur à oxyde métallique qui contient des oxydes de bismuth, de molybdène, de fer, de même qu'au moins un des éléments K, Rb, Cs, Cr, Mn ou P.

11. Procédé selon les revendications 1 à 10, caractérisé en ce qu'on chauffe le catalyseur à oxyde métallique à une température de 300 à 700 °C, avant l'étape a), en ajoutant des gaz contenant de l'oxygène moléculaire.
